# EUROPEAN PATENT SPECIFICATION

(11) **EP 0 716 595 B1**
(45) Date of publication and mention of the grant of the patent: **25.11.1998**
(21) Application number: 94926618.3
(22) Date of filing: 31.08.1994
(51) Int. Cl.: A61K 9/00, A61K 9/20

(54) **DISPENSER CONTAINING HYDROPHOBIC AGENT**
BEHÄLTER, DER EIN HYDROPHOBER ARZNEIMITTEL ENTHÄLT
DISTRIBUTEUR CONTENANT UN AGENT HYDROPHOBE

(30) Priority: 31.08.1993 US 114573
(43) Date of publication of application: 19.06.1996
(73) Proprietor: ALZA CORPORATION, Palo Alto California 94303-0802 (US)
(72) Inventor: DONG, Liang, C., Mountain View, CA 94043 (US); ESPINAL, Steven, D., Sunnyvale, CA 94086 (US); WONG, Patrick, S.-L., Palo Alto, CA 94306 (US); GUITTARD, George, V., Cupertino, CA 95014 (US); HAMEL, Lawrence, G., Mountain View, CA 94040 (US)
(74) Representative: Stebbing, Peter John Hunter
(86) International application number: US9409709
(87) International publication number: WO9506460

(56) References cited:
- US-A- 5 192 550
- CHEMICAL ABSTRACTS, vol. 100, no. 24, 11 June 1984, Columbus, Ohio, US; abstract no. 197699, HIRAKAWA, YOSHIYUKI, ET AL. 'Studies on development of the ultrafine size reduction method of slightly soluble medicinal crystals. V. Size reduction of phenytoin and phenobarbital' & YAKUGAKU ZASSHI, vol.104, no.1, 1984 pages 91 - 96

## Description

### FIELD OF THE INVENTION

The present invention pertains to the delivery of an active agent to an animal. More particularly, the invention is concerned with the controlled administration of a hydrophobic active agent, preferably a drug, to an animal over time, where the delivery device has a high loading of the hydrophobic agent.

### BACKGROUND OF THE INVENTION

Osmotic delivery devices for administering an active agent to a biological fluid environment of use are known in the art. Examples of such devices are disclosed in, e.g., U.S. Pat. Nos. 4,627,850; 4,663,148; 4,663,149;4,678,467; 4,692,326; 4,716,031; 4,814,180; 4,874,388; 5,023,088; and 5,126,142. While the prior art delivery devices usually work successfully for their intended purpose, the present inventors have observed that the devices often do not function well when the dispensed formulation contains a hydrophobic active agent in a very high loading, of 50% or greater. It has been found that when a formulation containing at least 50% hydrophobic agent is dispensed from the previous devices, the agent aggregates in the device when it is exposed to the fluid biological environment of use during release. Such agent aggregation is often the cause of uncontrolled and nonuniform release of the agent, and this in turn results in erratic release profiles and system malfunction.

While devices having a low loading of drug (of up to 40% or, more normally, less) can solve the problem of agent aggregation, this low loading creates a problem in those instances where it is desired to deliver a large amount of agent from the device. The amount of active agent necessary to be included in such a device would require a very large amount of formulation at a low dosing, making the device far too large and bulky to be useful as a practical matter as an implant or an oral dosage form, for example.

In the light of the above presentation, it will be appreciated by those versed in the dispensing art to which this invention pertains that a pressing need exists for a dosage form that can deliver a hydrophobic active agent to a biological environment of use in large amounts in a small volume while overcoming erratic delivery release profiles and system malfunction.

### SUMMARY OF THE INVENTION

The present invention is directed to a formulation for the delivery of hydrophobic active agents to a fluid environment of use while avoiding aggregation of the hydrophobic agents in the environment, the formulation comprising a hydrophobic active agent in an amount of 50 wt% (percent by weight) or greater, polyoxyethylene 40 stearate, and a disintegrant.

The present invention is also directed to an improvement in a fluid-activated delivery device wherein the delivery device includes a housing defining an internal compartment, an active agent formulation in the compartment, exit means in the housing for delivering the active agent formulation from the delivery device, a fluid-activated expandable driving member in the compartment, optionally a partition layer between the active agent formulation and the driving member, and optionally a lubricant subcoat; wherein the improvement comprises an active agent formulation comprising a hydrophobic active agent in an amount of 50 wt% or greater, polyoxyethylene 40 stearate, and a disintegrant.

### BRIEF DESCRIPTION OF THE DRAWINGS

FIG. **1** illustrates one embodiment of a device according to the present invention.

FIG. **2** illustrates another embodiment of a device according to the present invention.

FIGS. **3A** and **3B** are bar graphs showing the release rate profile of a gemfibrozil drug formulation of the present invention (FIG. **3A**) and of a prior art gemfibrozil formulation (FIG. **3B**).

FIGS. **4A**, **4B**, **4C**, and **4D** are bar graphs showing the release rate profile of a gemfibrozil drug formulation of the present invention (FIG. **4A**), and of gemfibrozil formulations that do not fall under the present invention (FIGS. **4B**, **4C**, and **4D**).

FIGS. **5A** and **5B** are bar graphs showing the release rate profile of a gemfibrozil drug formulation of the present invention (FIG. **5A**), and of a gemfibrozil formulation that does not fall under the present invention (FIG. **5B**).

FIG. **6** is a bar graph showing the release rate profile of a phenytoin drug formulation of the present invention.

FIG. **7** is a bar graph showing the release rate profile of a progesterone drug formulation of the present invention.

### DETAILED DESCRIPTION OF THE INVENTION AND PREFERRED EMBODIMENTS

It has now been discovered by the inventors that it is possible for hydrophobic active agents to be combined at a high agent loading (that is, in an amount of 50 wt% or greater) with a particular surfactant, polyoxyethylene 40 stearate, and also with a disintegrant. This high loading provides the advantage of obtaining a maximum amount of agent in a minimum amount of carrier to give fluid-activated delivery devices of a small size that are convenient for use as implants or oral dosage forms. The combination surprisingly maintains the hydrophobic active agent formulation in a non-aggregated form when the formulation is exposed to the fluid environment of use, which provides controlled and uniform release of the agent, and this in turn results in the alleviation of erratic release profiles and system malfunction.

The term "active agent" as used herein describes any beneficial agent or compound that can be delivered by a device according to this invention to produce a beneficial or useful, including a therapeutic, result. The term includes medicines or drugs, such as inorganic or organic drugs, biocides, sterilization agents, food supplements, nutrients, vitamins, sex sterilants, fertility inhibitors and fertility promoters. The agents include drugs which act on the peripheral nerves, adrenergic receptors, cholinergic receptors, the skeletal muscles, the cardiovascular system, smooth muscles, the blood circulatory system, synoptic sites, neuroeffector junctional sites, endocrine and hormone systems, the immunological system, the reproductive system, the skeletal system, autocoid systems, the alimentary and excretory systems, the histamine system and the central nervous system. Suitable agents may be selected from, for example, proteins, enzymes, hormones, polynucleotides, nucleoproteins, polysaccharides, glycoproteins, lipoproteins, polypeptides, steroids, analgesics, local anesthetics, antibiotic agents, anti-inflammatory corticosteroids, ocular drugs and synthetic analogs of these species.

The active agents included in the present invention are hydrophobic, that is they are insoluble or only slightly soluble in water. Any hydrophobic active agent which falls within this definition can be used in the present invention. Such active agents are known in the art or they can be determined without undue experimentation by reference to, for example, *The Merck Index or The Physician's Desk Reference* or other similar pharmaceutical or chemical reference books. Presently preferred hydrophobic active agents for use in this invention are progesterone, phenytoin, and gemfibrozil.

It is to be understood that more than one hydrophobic active agent may be incorporated into the active agent formulation of this invention, and that the use of the term "agent" in no way excludes the use of two or more such active agents. The terms "active agent" and "drug" are used interchangeably herein.

The active agent can be present in this invention in a wide variety of chemical and physical forms, such as uncharged molecules, molecular complexes, and pharmaceutically acceptable acid addition and base addition salts. Derivatives such as esters, ethers and amides can be used. The amount of active agent or drug present in a device according to the invention generally can be from about 0.05 ng to 10 g or more. The devices of the invention generally dispense from 0.1 to 200 mg/hr.

The hydrophobic active agent is present in the invention in a therapeutically effective amount; that is, in an amount that is necessary to provide a desired therapeutic, usually beneficial effect. The active agent will be present in the agent formulation at a loading of 50-99 wt%, preferably at least 60 wt%, and can be in an amount of 85 wt% or greater.

Surprisingly, it has now been found that when a hydrophobic active agent in an amount of at least 50 wt% is combined with a particular surfactant, polyoxyethylene 40 stearate, and also with a disintegrant, that the hydrophobic agent formulation does not aggregate when exposed to a fluid environment, such as an animal body. As a result, the functioning of the delivery system and the delivery of the active agent is greatly improved, providing a continuous and non-erratic agent release profile.

The amount of polyoxyethylene 40 stearate (MYRJ® 52-S, ICI Americas, Inc.) in the formulation of the present invention will be from 1 wt% to 25 wt%, preferably from 3 wt% to 20 wt%.

The disintegrant for use in the present invention is selected from any natural chemical entities or their derivatives or any synthetic chemicals that can promote rapid disintegration of a solid hydrophobic dosage form. Disintegrants useful herein include, but are not limited to, crospovidone (cross-linked polyvinylpyrrolidone), croscarmellulose sodium (Ac-Di-Sol®, FMC Corporation), sodium starch glycolate (Explotab®, Edward Mendell Co.), alginates, kappa-carrageenan, soya polysaccharides, cross-linked gelatin, ion-exchange resins, cyclodextrin polymers, cross-linked casein, and low substituted hydroxypropylcellulose. Presently preferred disintegrants for use in this invention are crospovidone and croscarmellulose sodium. The disintegrant is present in an amount of from 5 wt% to 30 wt%, preferably from 8 wt% to 20 wt%.

While the above surfactant and disintegrants are known in the art, it has not previously been known to use these two specific ingredients together in combination with a hydrophobic active agent at a high drug loading of at least 50 wt% to provide an improved formulation for avoiding aggregation of the agent to give improved delivery from a fluid-activated delivery device.

The hydrophobic active agent formulation of the present invention may optionally comprise additional inert ingredients, such as fillers, dyes, binders, lubricants, stabilizers, and the like, as are known in the art.

The formulation of the present invention is of particular use in fluid-activated delivery devices for delivering a hydrophobic active agent to a fluid environment of use, such as the body of an animal. Fluid-activated delivery devices are generally known in the art. The present invention is directed to an improved delivery device which includes a housing defining an internal compartment, a hydrophobic active agent formulation in the compartment, exit means in the housing for delivering the active agent formulation from the delivery device, a fluid-activated expandable driving member in the compartment, optionally a partition layer between the active agent formulation and the driving member, and optionally a lubricant subcoat; wherein the improvement comprises an active agent formulation comprising a hydrophobic active agent in an amount of 50 wt% or greater, polyoxyethylene 40 stearate, and a disintegrant.

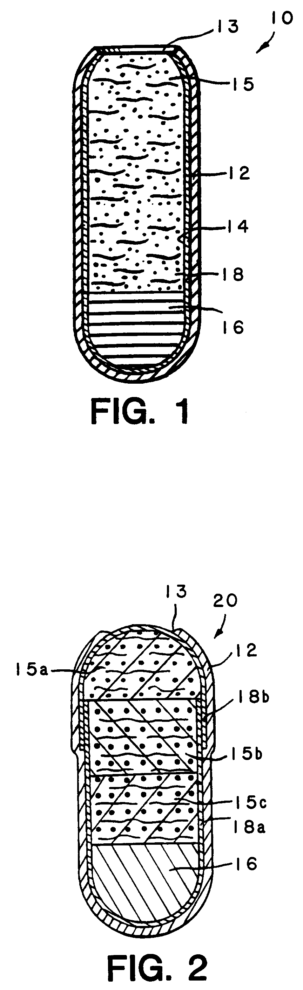

FIG. **1** illustrates one embodiment of a delivery device according to the invention. In the following discussion, like reference numerals refer to like elements in the Figures, which Figures are not drawn to scale. In FIG. **1**, device **10** comprises a rigid semipermeable housing **12** in the shape of a capsule which surrounds an internal compartment **14**. Semipermeable materials useful as housing **12** are known in the art, examples of which are discussed in U.S. Pat. No. 4,874,388, the entire disclosure of which is incorporated herein by reference.

An active agent formulation **15** according to the present invention is present in one portion of compartment **14**, formulation **15** comprising a hydrophobic active agent in an amount of at least 50 wt%, the surfactant polyoxyethylene 40 stearate, and a disintegrant. Exit port **13** is present in that portion of housing **12** that is adjacent to the active agent formulation **15** for providing a passageway between internal compartment **14** and the external environment.

Device **10** includes a driving member **16** that is fluid-activated. Many different types of fluid-activated driving components are known in the art, examples of which are discussed in detail in U.S. Pat. No. 4,874,388, incorporated previously herein by reference, and may include a water-swellable composition, an osmotically effective solute, an elementary osmotic pump or a gas-generating composition, for example. A moveable inert partition layer (not shown) may optionally be present to separate the active agent formulation **15** from the driving member **16**, and in a preferred embodiment the partition is substantially impermeable to the passage of fluid.

Device **10** optionally includes a subcoat **18** which is water-soluble and dissolves in the fluid environment of use. Subcoat **18** is not essential to the invention but when it is present, in its hydrated state it provides lubricant means for assisting the active agent formulation **15** to move within compartment **14** towards and out of the exit port **13** as a result of the expanding action of driving member **16**. Lubricant subcoat **18** may be chosen from water-soluble, hydratable materials such as gelatin, commercial materials having gelatin as one component, hydroxyethylcellulose, hydroxypropylcellulose, hydroxypropylmethylcellulose, and their derivatives, and the like.

FIG. **2** illustrates another embodiment of the present invention. Device **20** comprises a semipermeable wall **12**, an exit port **13**, an internal compartment **14**, and a fluid-activated driving member **16**. Device **20** has a hydrophobic active agent formulation according to the invention, the formulation being present as three tablets **15a**, **15b**, and **15c**. This configuration is merely illustrative, and the device may have agent tablets of a number other than the number shown in FIG. **2**.

Device **20** has a lubricant subcoat **18** which initially consists of two parts, a body portion **18a** and a cap portion **18b** to form a capsule. Capsule **18** is useful in the manufacture of device **20** for initially accepting and containing active agent formulation tablets **15a**, **15b**, and **15c** and driving member **16** prior to addition of the semipermeable housing **12**. When device **20** is placed in a fluid environment, capsule **18** hydrates to form a continuous lubricant subcoat.

While FIGS. **1** and **2** illustrate various delivery devices according to the present invention, it is to be understood that these dosage forms are not to be construed as limiting the invention, as the delivery device can take other shapes, sizes and forms for delivering the hydrophobic active agent formulation of the invention to a biological environment of use. The delivery device may be used to deliver a hydrophobic active agent to animals including warm-blooded animals, mammals and humans. The delivery device can be used in hospitals, clinics, nursing homes, veterinary clinics, farms, zoos, laboratories, and other fluid environments of use. The presently preferred environment of use comprises the gastrointestinal tract of a human. However, the devices are not restricted to such use. The environment of use can comprise a body cavity such as the peritoneum, vagina, or rumen (where a density element is then utilized for maintaining the device in the rumen). The device may also be utilized as a subcutaneous implant. A single dispensing device or several dispensing devices can be administered to a subject during a therapeutic program.

### EXAMPLE 1

A drug delivery device corresponding to the device in FIG. **2** was manufactured for dispensing gemfibrozil to the gastrointestinal tract, as follows.

A drug-layer formulation having a composition of 85.0 wt% gemfibrozil, 8.0 wt% croscarmellulose sodium (Ac-Di-Sol®, FMC Corporation), 3.0 wt% polyoxyethylene 40 stearate (MYRJ® 52-S), 3.0 wt% hydroxypropylmethylcellulose (HPMC E5), 0.5 wt% colloidal silicon dioxide and 0.5 wt% magnesium stearate was prepared by first passing each of the first four ingredients through a 40-mesh screen and then blending them together. Ethanol was slowly added into the blend and mixed with the blend until even consistency of a wet mass was attained. The wet mass was passed through a screen and then dried in an oven. Then, the silicon dioxide and magnesium stearate were added to the granules and blended. The drug-layer granules were compressed into single-layer flat-flat tablets each weighing 212 mg and flat-round tablets each weighing 282 mg, using a press with a 7 mm (17/46-inch) round punch.

An osmotic driving member tablet having a composition of 58.75 wt% sodium carboxymethylcellulose (7H4F), 30.0 wt% sodium chloride, 5.0 wt% HPMC E5, 1.0 wt% red ferric oxide, 5.0 wt% hydroxypropylcellulose (EF) and 0.25 wt% magnesium stearate was prepared by first passing each of the first four ingredients through a 40-mesh screen and then blending them together in a fluid-bed granulator and spraying the blend with the hydroxypropylcellulose in solution in purified water until homogeneous granules formed. These granules were passed through an 8-mesh screen and mixed with the magnesium stearate. The osmotic granules were compressed into single-layer flat-round tablets each weighing 250 mg, using a press with a 7 mm (17/46-inch) round punch.

To assemble the device, one osmotic tablet was placed in the bottom of one piece of a two-piece size "0" elongated gelatin capsule. Two flat-flat drug-layer tablets were placed in the capsule on top of the osmotic tablet. One flat-round drug-layer tablet was placed in the bottom of the other portion of the gelatin capsule and the two capsule pieces were fitted tightly together. The capsule was then coated with a semipermeable wall, the wall composition comprising 75.0 wt% cellulose acetate, having an acetyl content of 39.8%, and 25.0 wt% polyethylene glycol, having a molecular weight of 3,350. The wall-forming composition was dissolved in acetone/methanol (80/20, wt/wt) mixed solvent to make a 4% solid solution. The wall composition was sprayed onto the capsule to give a membrane weight of about 100 mg. Finally, an exit orifice (155 mil or 190 mil) was cut mechanically through the semipermeable membrane wall on the drug-layer end of the capsule. The residual solvent was removed by drying the device at 30°C and 50% humidity for 3 days.

The devices were tested for release characteristics by placing them in artificial intestinal fluid (AIF). They were removed and placed in new AIF at hourly intervals and the previous fluid was tested for the presence of gemfibrozil. The devices were found to deliver gemfibrozil at an average rate of 90.0 mg/hr with 90% of the drug released in 6 hrs.

### EXAMPLE 2

Following the procedures of Example 1, a gemfibrozil device was prepared having the following composition.

The gemfibrozil formulation was 66.7 wt% gemfibrozil, 0.5 wt% magnesium stearate, 5.0 wt% MYRJ 52-S,10.0 wt% Ac-Di-Sol, 12.8 wt% sodium phosphate and 5.0 wt% HPMC E-5. The final weight of the gemfibrozil formulation in the device was 600 mg.

The osmotic driving member composition was 58.5 wt% sodium carboxymethyl cellulose 7H4F, 35.0 wt% sodium chloride, 6.0 wt% HPMC E-5, 0.25 wt% ferric oxide and 0.25 wt% magnesium stearate. The final weight of the composition in the device was 300 mg.

The semipermeable membrane wall was 70.0 wt% cellulose acetate 398-10 (39.8% acetyl content) and 30.0 wt% polyvinyl pyrrolidone (PVP K29-32). The final weight of the membrane wall was 74.6 mg. The exit orifice was 190 mils.

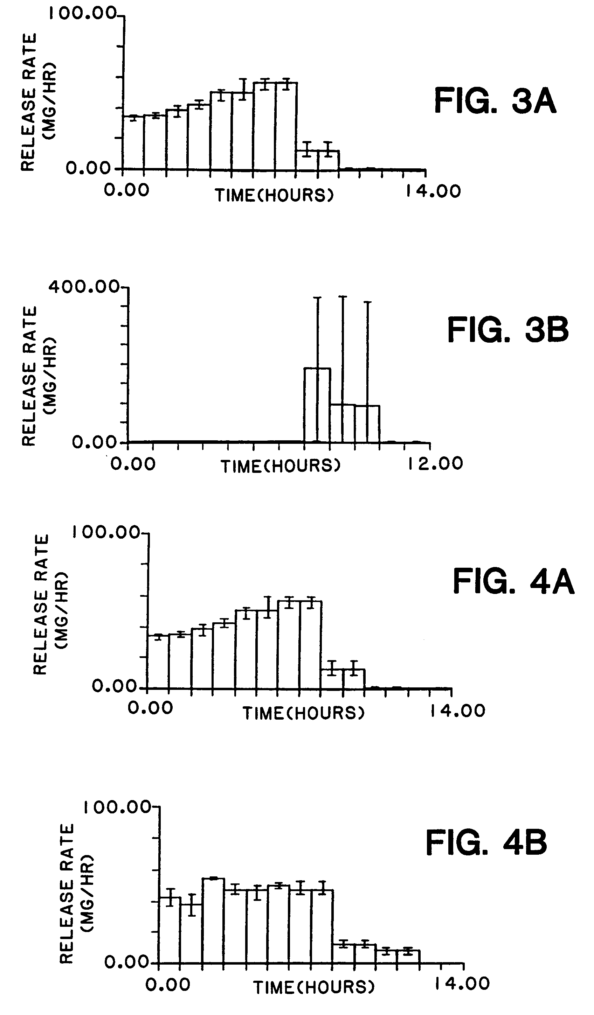

These devices were tested for gemfibrozil release in artificial gastric fluid (AGF), following the procedures of Example 1. The release rate profile is shown in FIG. **3A** (n=4).

For comparison purposes, a device having a prior art gemfibrozil formulation, without MYRJ 52-S or croscarmellulose, was prepared, the composition of the gemfibrozil formulation being 66.7 wt% gemfibrozil, 0.5 wt% magnesium stearate, 27.8 wt% sodium phosphate, and 5.0 wt% HPMC 5-E. The devices were tested for gemfibrozil release, as above, and the release rate profile is shown in FIG. **3B** (n=4).

### EXAMPLE 3

Following the procedures of Example 1, gemfibrozil-containing devices were prepared having the formulations under Table A:

**TABLE A**

| | Gemfibrozil | Mg Stearate | MYRJ | Ac-Di-Sol | Na2PO3 | HPMC | N80 Polyox |
|---|---|---|---|---|---|---|---|
| Device A (Ex. 2) | 66.7 | 0.5 | 5.0 | 10.0 | 12.8 | 5.0 | --- |
| Device B | 66.7 | 0.5 | --- | 10.0 | 7.8 | 5.0 | 10.0 |
| Device C | 66.7 | 0.5 | 5.0 | --- | 12.8 | 5.0 | 10.0 |
| Device D | 66.7 | 0.5 | --- | --- | 17.8 | 5.0 | 10.0 |

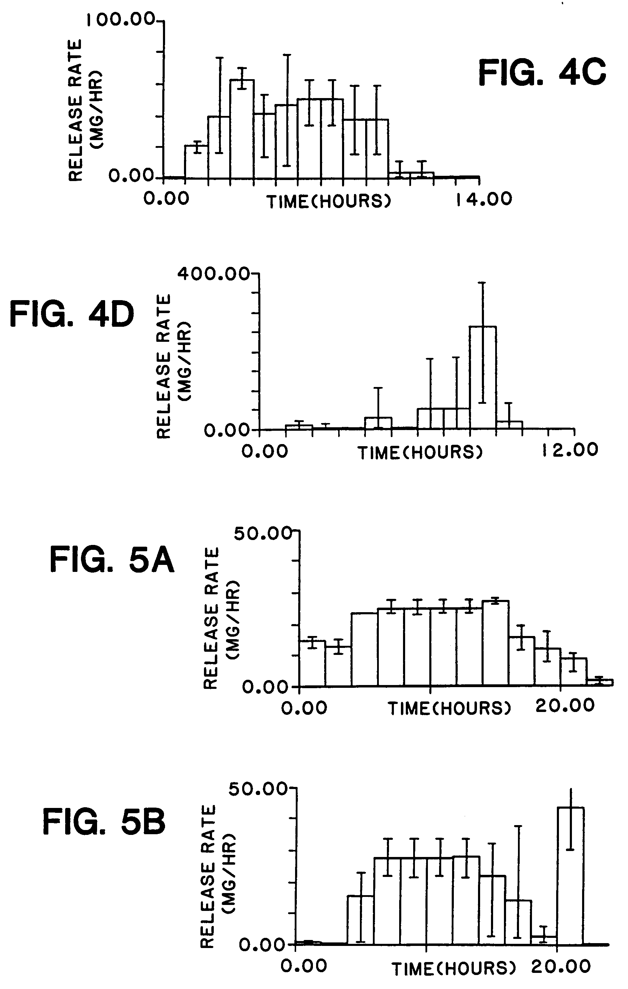

For comparison purposes, the devices were tested for gemfibrozil release against the gemfibrozil device of Example 2, in AGF. The release rate profiles are shown in FIG. **4A** (formulation A, having surfactant and disintegrant; same graph as in FIG. 3A; n=4), FIG. **4B** (formulation B, no surfactant; n=3), FIG. **4C** (formulation C, no disintegrant; n=4) and FIG. **4D** (formulation D, no surfactant or disintegrant; n=4).

### EXAMPLE 4

Following the procedures of Example 1, gemfibrozil-containing devices were prepared having the formulations under Table B:

**TABLE B**

| | Gemfibrozil | Mg Stearate | MYRJ | Ac-Di-Sol | HPMC | N10 Polyox |
|---|---|---|---|---|---|---|
| Device A | 66.7 | 0.5 | 5.0 | 12.8 | 5.0 | 10.0 |
| Device B | 66.7 | 0.5 | --- | 12.1 | 5.0 | 15.7 |

For comparison purposes, the devices were tested for gemfibrozil release in AGF. The release rate profiles are shown in FIG. **5A** (formulation A, having surfactant and disintegrant; n=3) and FIG. **4B** (formulation B, no surfactant; n=3).

### EXAMPLE 5

A phenytoin-containing device according to the present invention and corresponding to the device of FIG. **1** was prepared, generally following the procedures of Example 1.

The composition of the phenytoin drug tablet was 60.0 wt% phenytoin, 16.0 wt% sorbitol, 10.0 wt% MYRF 52-S, 10.0 wt% Ac-Di-Sol, 1.5 wt% magnesium stearate and 2.5 wt% PVP K29-32. The phenytoin composition was pressed into a single-layer elongated flat-round tablet weighing 460.0 mg.

The composition of the osmotic driving member tablet was the same as in Example 1. The final weight of the driving member in the device was 184.0 mg.

To assemble the device, 460 mg of the above phenytoin formulation and 184 mg of the osmotic composition were pressed together to form a bilayer capsule, using either a laboratory Carver press or a vertical compressing machine. A lubricant laminate subcoat of 95.0 wt% Natrosol and 5.0 wt% PEG 3350 was then spray-coated onto the bilayer drug/osmotic capsule. The laminate-coated capsule was dried and then coated with an outer semipermeable wall. The semipermeable membrane composition was 85.0 wt% cellulose acetate 398-10 and 15.0 wt% PEG 3350. The final weight of the membrane encasing the device was 49.0 mg. An exit orifice of 190 mils was then cut into the wall at the drug end of the capsule.

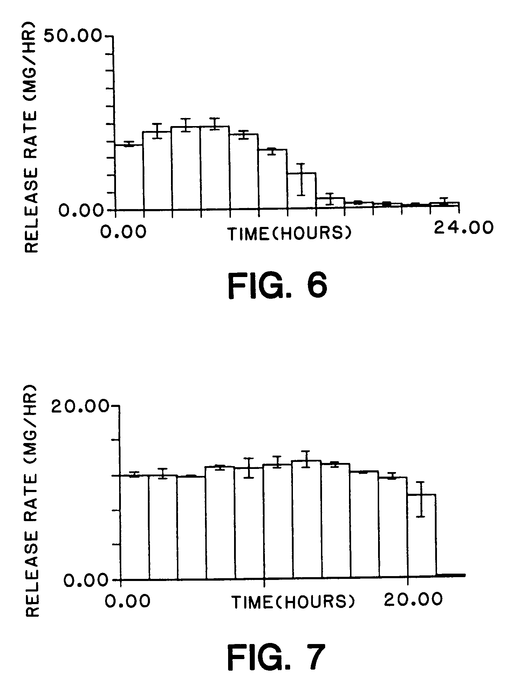

These devices were tested for phenytoin release in AIF, following the procedures of Example 1. The release rate profile is shown in FIG. **6** (n=5).

### EXAMPLE 6

A progesterone-containing device according to the present invention was prepared, generally following the procedures of Example 1 and corresponding to the device of FIG. **1**.

The composition of the progesterone drug tablets were 60.0 wt% progesterone, 9.5 wt% polyethylene oxide (Polyox® N10), 12.0 wt% MYRJ 52-S, 15.0 wt% cross-linked polyvinylpyrrolidone (XL-10; crospovidone), 0.5 wt% magnesium stearate and 3.0 wt% HPMC E-5.

The composition of the osmotic driving member tablet was the same as in Example 1.

To assemble the device, following the procedures of Example 4, 500 mg of the above progesterone formulation and 250 mg of the osmotic composition were compressed together to form a bilayer capsule-shaped tablet. A lubricant laminate subcoat (43.4 mg) of 95.0 wt% Natrosol and 5.0 wt% PEG 3350 was coated onto the drug/osmotic bilayer capsule prior to coating with the semipermeable membrane. The semipermeable membrane composition was 90.0 wt% cellulose acetate 398-10 and 10.0 wt% PEG 3350. The final weight of the membrane encasing the device was 85.0 mg. The exit orifice was 190 mils.

These devices were tested for progesterone release in AIF, following the procedures of Example 1. The release rate profile is shown in FIG. **7** (n=3).

### EXAMPLE 7

A gemfibrozil-containing device was prepared following the procedures of Example 1. The drug-layer and osmotic-layer compositions of the device were the same as those in Example 1. The semipermeable wall composition was 85.0 wt% cellulose acetate 398-10 and 15.0 wt% PEG 3350, and weighed 125 mg.

The devices were tested for gemfibrozil release in AIF, and they delivered the drug at an average rate of 30 mg/hr, with 90% of the drug released in 18 hrs.

### EXAMPLE 8

A different gemfibrozil-containing device was prepared following the procedures of Example 1. The composition of the osmotic layer is identical to that in Example 1, and the osmotic tablet weighed 300 mg. The semipermeable wall membrane composition was also identical to that of Example 6 and weighed 150 mg in the device of this example.

The gemfibrozil drug-layer formulation had a composition of 66.7 wt% gemfibrozil, 14.3 wt% croscarmellulose sodium, 5.0 wt% polyoxyethylene 40 stearate, 9.5 wt% polyethylene oxide (Polyox N10), 3.0 wt% HPMC 35, 1.0 wt% colloidal silicon dioxide, and 0.5 wt% magnesium stearate. The drug formulation in the device of this example was composed of one flat-round tablet and two flat-flat tablets, each weighing 133 mg.

The devices were tested for gemfibrozil release in AIF, and they delivered the drug at an average rate of 20 mg/hr, with 90% of the drug released in 18 hrs.

### EXAMPLE 9

A progesterone-containing device according to the present invention was prepared, following the procedures of Example 1.

The composition of the progesterone drug tablets were 60.0 wt% progesterone, 16.5 wt% MYRJ 52-S, 20.0 wt% Aci-Di-Sol, 3.0 wt% HPMC E-5 and 0.5 wt% magnesium stearate. The total weight of the drug tablets in the device was 500 mg.

The composition of the osmotic driving member tablet was the same as in Example 1. The weight of the driving member in the device was 250 mg.

To assemble the device, one osmotic tablet and two drug tablets (one flat-flat tablet weighing 220 mg and one flat-round tablet weighing 280 mg) were placed in a "0"-sized gelatin capsule. The capsule was coated with a semipermeable wall membrane composition identical to that of Example 6 and weighing 100 mg in the device of this example.

The devices were tested for progesterone release in AIF, and they delivery the drug at an average rate of 13.5 mg/hr with 90% of the drug released in 20 hr.

## Claims

1. A hydrophobic active agent formulation comprising:
50-99 wt% of a hydrophobic active agent;
1-25 wt% of polyoxyethylene 40 stearate; and
5-30% wt% of a disintegrant, selected from those disintegrants that promote rapid disintegration of a solid hydrophobic dosage form.

2. A formulation according to claim 1 wherein the hydrophobic active agent is selected from gemfibrozil, phenytoin and progesterone.

3. A formulation according to claim 1 or claim 2 wherein the disintegrant is selected from croscarmellulose sodium and crospovidone.

4. A formulation according to claim 1 wherein the hydrophobic active agent is gemfibrozil and the disintegrant is croscarmellulose sodium.

5. A formulation according to claim 1 wherein the hydrophobic active agent is phenytoin and the disintegrant is croscarmellulose sodium.

6. A formulation according to claim 1 wherein the hydrophobic active agent is progesterone and the disintegrant is selected from croscarmellulose sodium and crospovidone.

7. A formulation according to any preceding claim which comprises at least 60 wt% hydrophobic active agent, 3-20 wt% polyoxyethylene 40 stearate, and 8-20 wt% disintegrant.

8. A delivery device (10-Fig.1) for dispensing a hydrophobic active agent to a fluid environment of use, the delivery device comprising a housing (12) defining an internal compartment (14), a hydrophobic active agent formulation (15) in the compartment, exit means (13) in the housing for delivering the active agent formulation from the delivery device, and a fluid-activated driving member (16) in the compartment; characterized by an active agent formulation comprising 50-99 wt% of a hydrophobic active agent, 1-25 wt% of polyoxyethylene 40 stearate, and 5-30 wt% of a disintegrant selected from those disintegrants that promote rapid disintegration of a solid hydrophobic dosage form.

9. A delivery device according to claim 8 wherein the disintegrant is selected from croscarmellulose sodium and crospovidone.

10. A delivery device according to either of claims 8 or 9 wherein the hydrophobic active agent is selected from the gemifibrozil, phenytoin and progesterone.

11. A delivery device according to claim 8 wherein the hydrophobic active agent is gemifibrozil and the disintegrant is croscarmellulose sodium.

12. A delivery device according to claim 8 wherein the hydrophobic active agent is phenytoin and the disintegrant is croscarmellulose sodium.

13. A delivery device according to any of claims 8 to 12 comprises at least 60 wt% hydrophobic active agent, 3-20 wt% polyoxyethylene 40 stearate, and 8-20 wt% disintegrant.

14. A delivery device according to any of claims 8 to 13 which further comprises a partition layer between the active agent formulation and the driving member.

15. A delivery device according to any of claims 8 to 14 which further comprises a lubricant subcoat.

## Patentansprüche

1. Zubereitungsform bzw. Formulierung eines hydrophoben Wirkstoffs umfassend:
50-99 Gew.-% eines hydrophoben Wirkstoffs;
1-25 Gew.-% eines Polyoxyethylen 40-Stearats; und
5-30 Gew.% eines Zerfallmittels ausgewählt aus solchen Zerfallmitteln, die einen schnellen Zerfall einer festen hydrohoben Dosierungsform beschleunigen.

2. Zubereitungsform nach Anspruch 1, wobei der hydrophobe Wirkstoff ausgewählt ist aus Gemfibrozil, Phenytoin und Progesteron.

3. Zubereitungsform nach Anspruch 1 oder 2, wobei das Zerfallmittel ausgewählt ist aus Croscarmellulose-Natrium und Crospovidon.

4. Zubereitungsform nach Anspruch 1, wobei der hydrophobe Wirkstoff Gemfibrozil und das Zerfallmittel Croscarmellulose-Natrium ist.

5. Zubereitungsform nach Anspruch 1, wobei der hydrophobe Wirkstoff Phenytoin und das Zerfallmittel Croscarmellulose-Natrium ist.

6. Zubereitungsform nach Anspruch 1, wobei der hydrophobe Wirkstoff Progesteron ist und das Zerfallmittel ausgewählt ist aus Croscarmellulose-Natrium und Crospovidon.

7. Zubereitungsform nach einem der vorhergehenden Ansprüche, wobei diese mindestens 60 Gew.-% hydrophoben Wirkstoff, 3-20 Gew.% Polyoxyethylen 40-Stearat und 8-20 Gew.-% Zerfallmittel umfaßt.

8. Abgabevorrichtung (10 - Fig. 1) zum Dispensieren eines hydrophoben Wirkstoffs in eine Fluidumgebung bei Verwendung, wobei die Abgabevorrichtung ein Gehäuse (12), das eine innere Kammer (14) begrenzt, eine Zubereitungsform eines hydrohoben Wirkstoffs (15) in der Kammer, Austrittseinrichtungen (13) im Gehäuse zum Freisetzen der Wirkstoff-Zubereitungsform aus der Abgabevorrichtung und ein Fluid-aktiviertes Antriebselement (16) in der Kammer umfaßt, gekennzeichnet durch eine Wirkstoff-Zubereitungsform umfassend 50-99 Gew.-% eines hydrophoben Wirkstoffs, 1-25 Gew.-% eines Polyoxyethylen 40-Stearats, und 5-30 Gew.% eines Zerfallmittels, das einen schnellen Zerfall einer festen hydrohoben Dosierungsform beschleunigt.

9. Abgabevorrichtung nach Anspruch 8, wobei das Zerfallmittel ausgewählt ist aus Croscarmellulose-Natrium und Crospovidon.

10. Abgabevorrichtung nach entweder Anspruch 8 oder 9, wobei der Wirkstoff ausgewählt ist aus Gemfibrozil, Phenytoin und Progesteron.

11. Abgabevorrichtung nach Anspruch 8, wobei der hydrophobe Wirkstoff Gemfibrozil und das Zerfallmittel Croscarmellulose-Natrium ist.

12. Abgabevorrichtung nach Anspruch 8, wobei der hydrophobe Wirkstoff Phenytoin und das Zerfallmittel Croscarmellulose-Natrium ist.

13. Abgabevorrichtung nach einem der Ansprüche 8 bis 12, die mindestens 60 Gew.-% hydrophoben Wirkstoff, 3-20 Gew.% Polyoxyethylen 40-Stearat und 8-20 Gew.-% Zerfallmittel umfaßt.

14. Abgabevorrichtung nach einem der Ansprüche 8 bis 13, welche weiter eine Trennschicht zwischen der Wirkstoff-Zubereitungsform und dem Antriebselement umfaßt.

15. Abgabevorrichtung nach einem der Ansprüche 8 bis 14, welche weiter einen Gleitmittelüberzug umfaßt.

## Revendications

1. Formulation d'agent actif hydrophobe, comprenant :
- 50-99% en poids d'un agent actif hydrophobe ;
- 1-25% en poids de stéarate de polyoxyéthylène 40 ; et
- 5-30% en poids d'un désintégrant, choisi parmi les désintégrants qui favorisent une désintégration rapide d'une forme de dosage hydrophobe solide.

2. Formulation selon la revendication 1, dans laquelle l'agent actif hydrophobe est choisi parmi le gemfibrozil, la phénytoïne et la progestérone.

3. Formulation selon la revendication 1 ou la revendication 2, dans laquelle le désintégrant est choisi parmi la croscarmellulose sodique et la crospovidone.

4. Formulation selon la revendication 1, dans laquelle l'agent actif hydrophobe est le gemfibrozil, et le désintégrant est la croscarmellulose sodique.

5. Formulation selon la revendication 1, dans laquelle l'agent actif hydrophobe est la phénytoïne, et le désintégrant est la croscarmellulose sodique.

6. Formulation selon la revendication 1, dans laquelle l'agent actif hydrophobe est la progestérone, et le désintégrant est choisi parmi la croscarmellulose sodique et la crospovidone.

7. Formulation selon l'une quelconque des revendications précédentes, qui comprend au moins 60% en poids d'agent actif hydrophobe, 3-20% en poids de stéarate de polyoxyéthylène 40, et 8-20% en poids de désintégrant.

8. Dispositif de distribution (10 - Figure 1), pour distribuer un agent actif hydrophobe dans un milieu d'usage fluide, le dispositif de distribution comprenant une enveloppe (12) définissant un compartiment interne (14), une formulation (15) d'agent actif hydrophobe dans le compartiment, des moyens de sortie (13) dans l'enveloppe pour distribuer la formulation d'agent actif à partir du dispositif de distribution, et un élément d'entraînement (16), activé par un fluide, dans le compartiment, caractérise par une formulation d'agent actif comprenant 50-99% en poids d'un agent actif hydrophobe, 1-25% en poids de stéarate de polyoxyéthylène 40, et 5-30% en poids d'un désintégrant choisi parmi les désintégrants qui favorisent une désintégration rapide d'une forme de dosage hydrophobe solide.

9. Dispositif de distribution selon la revendication 8, dans lequel le désintégrant est choisi parmi la croscarmellulose sodique et la crospovidone.

10. Dispositif de distribution selon l'une des revendications 8 ou 9, dans lequel l'agent actif hydrophobe est choisi parmi le gemfibrozil, la phénytoïne et la progestérone.

11. Dispositif de distribution selon la revendication 8, dans lequel l'agent actif hydrophobe est le gemfibrozil, et le désintégrant est la croscarmellulose sodique.

12. Dispositif de distribution selon la revendication 8, dans lequel l'agent actif hydrophobe est la phénytoïne, et le désintégrant est la croscarmellulose sodique.

13. Dispositif de distribution selon l'une quelconque des revendications 8 à 12, dans lequel la formulation comprend au moins 60% en poids d'agent actif hydrophobe, 3-20% en poids de stéarate de polyoxyéthylène 40, et 8-20% en poids de désintégrant.

14. Dispositif de distribution selon l'une quelconque des revendications 8 à 13, qui comprend en outre une couche de séparation entre la formulation d'agent actif et l'élément d'entraînement.

15. Dispositif de distribution selon l'une quelconque des revendications 8 à 14, qui comprend en outre une sous-couche de lubrifiant.
